# EUROPÄISCHE PATENTSCHRIFT

(11) **EP 2 376 434 B1**
(45) Veröffentlichungstag und Bekanntmachung des Hinweises auf die Patenterteilung: **24.05.2017**
(21) Anmeldenummer: 09799248.1
(22) Anmeldetag: 10.12.2009
(51) Int. Cl.: C07C 251/70, C23C 18/12, H01L 21/335, H05K 3/12, H05K 3/14

(54) **FUNKTIONELLES MATERIAL FÜR GEDRUCKTE ELEKTRONISCHE BAUTEILE**
FUNCTIONAL MATERIAL FOR PRINTED ELECTRONIC COMPONENTS
MATÉRIAU FONCTIONNEL DESTINÉ À DES COMPOSANTS ÉLECTRONIQUES IMPRIMÉS

(30) Priorität: 09.01.2009 DE 102009004491
(43) Veröffentlichungstag der Anmeldung: 19.10.2011
(73) Patentinhaber: Merck Patent GmbH, 64293 Darmstadt (DE)
(72) Erfinder: KUEGLER, Ralf, Cambridge MA 02139 (US); KLYSZCZ, Andreas, 64291 Darmstadt (DE); RENKER, Sabine, Tokyo 160-0023 (JP); SCHNEIDER, Joerg J., 64342 Seeheim-Jugenheim (DE); HOFFMANN, Rudolf, 64291 Darmstadt (DE)
(86) Internationale Anmeldenummer: PCT/EP2009/008821
(87) Internationale Veröffentlichungsnummer: WO 2010/078907

(56) Entgegenhaltungen:
- WO-A2-2009/010142
- HILL, M.R. ET AL.: "Towards new precursors for zno thin films by single source CVD:the X-ray structures and precursor properties of zinc ketoacidoximates" INORGANICA CHIMICA ACTA, Bd. 358, 2005, Seiten 201-206, XP002574352
- DATABASE CA [Online] CHEMICAL ABSTRACTS SERVICE, COLUMBUS, OHIO, US; 1993, APBLETT, ALLEN W. ET AL: "Incorporation of radionuclides into mineral phases via a thermally unstable complexant ligand" XP002574350 gefunden im STN Database accession no. 119:258193 & APBLETT, ALLEN W. ET AL: "Incorporation of radionuclides into mineral phases via a thermally unstable complexant ligand" MATERIALS RESEARCH SOCIETY SYMPOSIUM PROCEEDINGS , 294(SCIENTIFIC BASIS FOR NUCLEAR WASTE MANAGEMENT XVI), 123-8 CODEN: MRSPDH; ISSN: 0272-9172, 1993,
- DATABASE CA [Online] CHEMICAL ABSTRACTS SERVICE, COLUMBUS, OHIO, US; 1988, KOJIMA, SHIGERU ET AL: "Preparation of iminoacetic acid derivatives as fungicides." XP002574351 gefunden im STN Database accession no. 110:71110 & JP 63 162673 A (NIPPON SODA CO., LTD., JAPAN) 6. Juli 1988 (1988-07-06)

## Beschreibung

Die Erfindung betrifft einen Gallium-, Ruthenium-, Magnesium-, Hafnium-, Indium- und/oder Zinn-haltigen Precursor für elektronische Bauteile sowie ein Herstellverfahren. Des Weiteren betrifft die Erfindung entsprechende gedruckte elektronische Bauelemente sowie dazu geeignete Herstellverfahren.

Für den Einsatz gedruckter Elektronik in Massenanwendungen (z.B. RFID (=Radio Frequency Identification) Chips auf Einzelverpackungen) ist die Verwendung von etablierten Massendruckverfahren wünschenswert. Generell bestehen gedruckte elektronische Bauteile und Systeme aus mehreren Materialkomponenten, wie Leiter für z.B. Kontaktierungen, Halbleiter z.B. als aktive Materialien, sowie Isolatoren z.B. als Sperrschichten.

Die Verfahren zur Herstellung bestehen zumeist aus einem Depositionsschritt, also dem Aufbringens des jeweiligen Materials auf ein Trägermaterial (Substrat), sowie aus einem darauf folgenden Prozessschritt, der die gewünschten Eigenschaften des Materials sicherstellt. Wünschenswert im Hinblick auf eine massentaugliche z.B. Rolle-zu-Rolle Verarbeitung ist die Verwendung flexibler Substrate (Folien). Bisherige Verfahren zur Herstellung gedruckter Schaltungen weisen intrinsische Vor- aber auch Nachteile auf:
- Konventionelle Technologie siehe WO 2004086289): Hier werden Hybride aus konventionellen Si-Logikbaustein und zusätzlicher strukturierter oder gedruckter Komponenten (z.B. Metallantenne im Falle RFID Chip) kostenintensiv zusammengefügt. Dieses Verfahren wird jedoch als zu aufwändig im Hinblick auf eine reale Volumenanwendung gesehen.
- Organische Materialien (siehe DE 19851703, WO 2004063806, WO 2002015264): Bei diesen Systemen handelt es sich um gedruckte Elektronikkomponenten auf Basis von Polymeren aus der flüssigen Phase. Diese Systeme zeichnen sich durch eine im Vergleich zu den vorher genannten Materialien (konventionelle Technologie) einfache Verarbeitung aus Lösungen aus. Als Prozessschritt ist hierbei nur die Trocknung des Lösungsmittels zu berücksichtigen. Allerdings ist die erreichbare Leistungsfähigkeit im Falle z.B. halbleitender oder leitender Materialien durch limitierende materialtypische Eigenschaften, wie z.B. der Ladungsträgerbeweglichkeit <10 cm² /Vs durch sog. Hopping-Mechanismen, beschränkt. Diese Beschränkung wirkt sich auf die Anwendungsmöglichkeiten aus: die Leitungsfähigkeit eines gedruckten Transistors erhöht sich mit reduzierter Ausdehnung des halbleitenden Kanals, der derzeit mit Massenverfahren nicht kleiner als ∼ 40 µm gedruckt werden kann. Eine weitere Beschränkung der Technologie stellt die Empfindlichkeit der organischen Komponenten gegenüber Umgebungsbedingungen dar. Diese bewirkt eine aufwändige Prozessführung während der Herstellung sowie gegebenenfalls eine verkürzte Lebensdauer der gedruckten Komponenten.
- Anorganische Materialien: diese Materialklasse hat generell durch unterschiedliche intrinsische Eigenschaften (z.B. Stabilität gegen UVbedingten Abbau) im Vergleich zu den organischen Materialien das Potential für eine erhöhte Leistungsfähigkeit bei Verwendung in gedruckter Elektronik.
In diesem Bereich können im Prinzip zwei unterschiedliche Ansätze zur Anwendung kommen:
i) Präparation aus der Gasphase ohne zusätzlichen Prozessschritt: in diesem Fall ist es möglich, sehr gut isolierende dünne Schichten zu erzeugen, allerdings limitiert die damit verbundene kostenintensive Vakuumtechnologie sowie das langsame Schichtwachstum eine Anwendung im Massenmarkt.
ii) Nasschemische Herstellung ausgehend von Precursormaterialien, wobei die Materialien aus der flüssigen Phase z.B. durch Aufschleudern oder Drucken aufgebracht werden (siehe US 6867081, US 6867422, US 2005/0009225). Teilweise werden auch Mischungen von anorganischen Materialien und organischer Matrix verwendet (siehe US 2006/0014365) Um eine durchgehende elektrische Eigenschaft der hergestellten Schicht zu gewährleisten, ist zumeist ein Prozessschritt erforderlich, der über ein Verdampfen des Lösungsmittels hinausgeht. In allen Fällen muss eine Morphologie mit ineinander übergehenden Bereichen erzeugt werden, wobei Precursoren aus der Nassphase zusätzlich in das gewünschte aktive Material überführt werden. Dadurch wird eine gewünschte Funktionalität erzeugt (im Falle von Halbleitern: hohe Ladungsträgerbeweglichkeit). Die Prozessierung erfolgt daher bei Temperaturen > 300°C, was allerdings eine Verwendung dieses Prozesses zur Folienbeschichtung verhindert.

Beispiele für die Verwendung eines Vorläufer- bzw. Precursormaterials für MgO wird z.B. in Stryckmans et al. Thin Solid Films 1996, 283, 17 (aus Magnesiumacetylacetonat oberhalb 260°C) oder Raj et al. Crystal Research and Technology 2007, 42, 867 (aus Magnesiumacetat ab 300°C in mehreren Stufen) beschrieben. In beiden Fällen wird dort zur Schichtabscheidung aus der Gasphase die Spray-Pyrolyse eingesetzt. Dabei werden die Lösungen auf ein aufgeheiztes Substrat (oberhalb 400°C) aufgespritzt und gegebenenfalls bei noch höherer Temperatur nachbehandelt. Die erforderlichen hohen Temperaturen verhindern eine Verwendung in Druckprozessen.

Ein Beispiel für die Verwendung eines löslichen ZrO₂- Precursormaterials wird in Ismail et al. Powder Technology 1995, 85, 253 (aus Zirkoniumacetylacetonat über mehrere Stufen zwischen 200 bis 600 °C) beschrieben.

Ein Beispiel für die Verwendung eines löslichen HfO₂ ist z.B. aus Zherikova et al. Journal of Thermal Analysis and Calorimetry 2008, 92, 729 (aus Hafniumacetylacetonat über mehrere Stunden zwischen T = 150 und 500°C) bekannt.

Beta-Diketonate, wie die Acetylacetonate von Zirkonium und Hafnium, werden in Schichtabscheidungen aus der Gasphase mittels Chemical Vapour Deposition (CVD) eingesetzt. Dabei werden die Verbindungen im Hochvakuum verdampft und auf aufgeheizten Substraten (oberhalb von 500°C) abgeschieden. Die Umwandlung zur oxidischen Keramik erfolgt über mehrere diskrete Zwischenstufen, die aber nicht als funktionales Material Verwendung finden. Damit sind hohe Temperaturen erforderlich, die eine definierte Umsetzung gewährleisten, was eine Verwendung in Druckprozessen verhindert.

Zur Herstellung von Indium-Zinn-Oxid (abgekürzt: "ITO" genannt) werden als Vorläufer beispielsweise Sole aus Zinn- und Indiumsalzen in Gegenwart von Aminen wie Ethanolamin verwendet (Prodi et al. Journal of Sol-Gel Science and Technology 2008, 47, 68). Die Umwandlung zum Oxid erfolgt hier bei Temperaturen oberhalb von 500-600°C. Die erforderlichen hohen Temperaturen verhindern eine Verwendung in Druckprozessen auf temperaturempfindlichen Substraten.

Von Interesse ist die Herstellung amorpher, halbleitender Oxidkeramiken (K. Nomura et al. Nature 2004, 432, 488-492; H.Hosono Journal of Non-Crystalline Solids 2006, 352, 851-858; T. Kamiya et al. Journal of Display Technology 2009, 5, 273-288). Dabei ist das Phasensystem Indium-Gallium-Zinn-Zink-Sauerstoff eingehend untersucht worden. Typische Beispiele sind Indium-Gallium-Zinkoxid (abgekürzt "IGZO"), sowie Zink-Zinnoxid (abgekürzt "ZTO"), aber auch Indium-Zink-Zinnoxid (abgekürzt "IZTO").

Die Abscheidung der halbleitenden Schichten erfolgt zumeist über die Gasphase, aber auch auf Lösungen basierende Prozesse sind bekannt. Die dabei eingesetzten Sole machen jedoch höhere Prozessierungstemperaturen erforderlich. Zink-Zinnoxid kann aus wasserfreiem Zinn(II)chlorid oder Zinn(II)acetat und Zinkacetathexahydrat in Gegenwart von Basen wie Ethanolamin erhalten werden. Die Umwandlung zum Oxid (unter Oxidation der Zinnkomponenten) erfolgt in Abhängigkeit von der Reaktionsführung bei Calcinierung bei mindestens 350°C (D. Kim et al. Langmuir 2009, 25, 11149-11154) oder 400-500°C (S.J. Seo et al. Journal of Physics D: Applied Physics, 2009, 42, 035106) an Luft. Indium-Zink-Zinnoxid wird aus wasserfreiem Indiumchlorid, Zinkchlorid und Zinn(II)chlorid in Ethylenglykol durch Reaktion mit Natronlauge und nachfolgender Calcinierung bei 600°C erhalten (D.H. Lee et al. Journal of Materials Chemistry 2009, 19, 3135-3137).

Aus M. R. Hill et. al., Inorganica Chimica Acta 358 (2005) 201 - 206 ist die Verwendung von Zink-Ketoacidooximaten zur Herstellung von Zinkoxid mit Hilfe eines CVD-Verfahrens bekannt.

Der Einsatz dieser herkömmlichen Precursoren zur Herstellung gedruckter Schaltungen ist in ihrer Anwendbarkeit in der Volumenproduktion einer Massendruckanwendung beschränkt.

In A. W. Apblett et. al., Mat. Res. Soc. Symp. Proc. Vol 294 (1993) 123 - 128, sind Brenztraubensäure-Metalloximate verschiedener Metalle beschrieben, die sich zur Herstellung spezieller Keramiken eignen, in denen radioaktiver Abfall gebunden werden soll.

JP-B-2556317 beschreibt Halogen-haltige Iminoessigsäurederivate und deren Metallsalze, die zur Herstellung von Pflanzenschutzmitteln verwendet werden.

Aufgabe der vorliegenden Erfindung war es daher, anorganische Materialien zur Verfügung zu stellen, deren dielektrische, halbleitende sowie leitende Eigenschaften einerseits durch die Materialzusammensetzung und andererseits durch das Herstellverfahren der gedruckten Materialien einstellbar sind. Dazu sollen Materialsysteme entwickelt werden, die die Vorteile anorganischer Materialien bewahren. Das Material soll aus der Nassphase durch ein Druckverfahren verarbeitet werden können. Mit einem Verfahrensschritt, der nur einen geringen Energieeintrag erfordert, soll die jeweils gewünschte elektronische Leistungsfähigkeit des Materials auf planaren, sowie flexiblen Substraten dargestellt werden.

Jetzt wurde überraschend ein Verfahren entwickelt, bei dem ein neuartiges metallorganisches Precursormaterial hergestellt, auf Oberflächen aufgebracht und im Anschluss daran bei niedrigen Temperaturen in ein dielektrisch aktives, d.h. isolierendes sowie auch in ein elektrisch halbleitendes oder leitendes Material umgewandelt wird. Die dabei erzeugten Schichten zeichnen sich durch Oberflächeneigenschaften aus, die für einen Druckprozess vorteilhaft sind.

Gegenstand der vorliegenden Erfindung ist somit ein Precursor zum Beschichten von elektronischen Bauteilen, gekennzeichnet dadurch, dass er einen metallorganischen Gallium-, Ruthenium-, Magnesium-, Hafnium- Indium- und/oder Zinnkomplex sowie deren Gemische enthält, der mindestens einen Liganden aus der Klasse der Oximate enthält.

Bei geeigneter Reaktionsführung können die Precursoren auch alkalimetallfrei hergestellt werden. Dies kann für den Einsatz in elektronischen Bauteilen vorteilhaft sein, da sich alkalimetallhaltige Rückstände negativ auf die elektronischen Eigenschaften auswirken können. Diese Elemente können als Fremdatome im Kristall die Eigenschaften ungünstig beeinflussen.

In einer bevorzugten Ausführungsform ist der Precursor druckfähig und liegt in Form einer Drucktinte oder Druckpaste zum Beschichten von gedruckten Feldeffekttransistoren (FET), vorzugsweise Dünnschichttransistoren (TFT) vor.

Unter dem Begriff "druckfähiger Precursor" ist ein Vorläufermaterial zu verstehen, das aufgrund seiner Materialeigenschaften aus der Nassphase durch ein Druckverfahren verarbeitet werden kann. Während des Druckprozesses wird eine Drucktinte oder Druckpaste aus einem Vorratsbehälter druckverfahrensabhängig auf ein Substrat transportiert.

Daher muss das Vorläufermaterial in eine solche Drucktinte oder Druckpaste überführbar sein, die für den Druckprozess geeignete Viskosität und Stabilität während des Druckprozesses besitzt, sowie geeignete Benetzbarkeit und Haftung auf dem Substrat aufweist. Erfahrungsgemäß sind für unterschiedliche Druckverfahren verschiedene Viskositätsbereiche der Farbe oder Paste bevorzugt; z.B. für Inkjet-Druck (thermisch) 1-5 mPa·s, Inkjet-Druck (piezoelektrisch) 5-20 mPa·s, Tiefdruck 50-200 mPa·s, Flexodruck 50-500 mPa·s, Siebdruck 2000-40000 mPa·s.

Wie oben schon beschrieben, enthält der Precursor einen metallorganischen Gallium-, Ruthenium-, Magnesium-, Hafnium- Indium- und/oder Zinnkomplex mit mindestens einem Liganden aus der Klasse der Oximate. Erfindungsgemäß bevorzugt ist es, wenn die Liganden des Gallium-, Ruthenium-, Magnesium-, Hafnium-, Indium- und/oder Zinnkomplexes ein 2-(Methoxyimino)alkanoat, 2-(Ethoxyimino)alkanoat oder 2-(Hydroxyimino)alkanoat enthalten. Die Liganden werden durch Kondensation von alpha-Ketosäuren bzw. Oxocarbonsäuren mit Hydroxylaminen oder Alkylhydroxylaminen in Gegenwart von Basen in wässriger oder methanolischer Lösung synthetisiert.

Die Precursoren bzw. Gallium-, Ruthenium-, Magnesium-, Hafnium-, Indium- und/oder Zinnkomplexe bilden sich bei Raumtemperatur durch Umsetzung einer Oxocarbonsäure mit mindestens einem Hydroxyl- oder Alkylhydroxylamin in Gegenwart einer Base, wie z.B. Tetraethylammoniumhydrogencarbonat oder Natriumhydrogencarbonat und anschließender Zugabe eines anorganischen Gallium-, Ruthenium-, Magnesium-, Hafnium-, Indium- und/oder Zinnsalzes wie z.B. Galliumnitrat-Hexahydrat, Rutheniumtrichloridhexahydrat, Magnesiumnitrat-Hexahydrat, Hafniumoxochlorid-Octahydrat, wasserfreies Indiumchlorid und/oder Zinnchlorid-Pentahydrat. Alternativ dazu kann eine Oxcarbonsäure in Gegenwart von mindestens einem Hydroxyl- oder Alkylhydroxylamin mit einem Hydroxocarbonat von Magnesium oder Hafnium, wie zum Beispiel Hydromagnesit Mg₅(CO₃)₄(OH)₂•4H₂O, umgesetzt werden. Als Oxocarbonsäure können alle Vertreter dieser Verbindungsklasse eingesetzt werden. Bevorzugt werden aber Oxoessigsäure, Oxopropionsäure oder Oxobuttersäure eingesetzt.

Die thermische Umwandlung des Gallium-, Magnesium- oder -Hafnium komplex-Precursors in die funktionelle Galliumoxid-, Magnesiumoxid-oder Hafniumoxid schicht mit isolierenden Eigenschaften bzw. des Rutheniumkomplex-Precursors in eine Schicht aus Rutheniumoxid erfolgt bei einer Temperatur ≥ 80 °C. Bevorzugt liegt die Temperatur zwischen 150 und 200 °C.
Die thermische Umwandlung der Indium- und Zinnkomplex-Precursoren in die funktionelle Indiumzinnoxid-Schicht mit leitenden Eigenschaften erfolgt bei einer Temperatur ≥ 150 °C. Bevorzugt liegt die Temperatur zwischen 150 und 250 °C.
Die thermische Umwandlung der Indium-, Gallium- und Zinkkomplex-Precursoren in die funktionelle Indium-Gallium-Zinkoxid-Schicht mit halbleitenden Eigenschaften erfolgt bei einer Temperatur ≥ 150 °C. Bevorzugt liegt die Temperatur zwischen 150 und 250 °C.
Die thermische Umwandlung der Zink- und Zinnkomplex-Precursoren in die funktionelle Zink-Zinnoxid-Schicht mit halbleitenden Eigenschaften erfolgt bei einer Temperatur ≥ 150 °C. Bevorzugt liegt die Temperatur zwischen 150 und 250 °C.

Die Umwandlung des Gallium-, Magnesium- oder Hafnium komplex-Precursors in die funktionelle Gallium-, Magnesium- oder Hafnium oxidschicht mit isolierenden Eigenschaften bzw. die Umwandlung des Rutheniumkomplex-Precursors in Rutheniumoxid bzw. die Umwandlung der Indium- und Zinnkomplex-Precursoren in die funktionelle Indiumzinnoxid-Schicht mit leitenden Eigenschaften bzw. die Umwandlung der Indium-Gallium-Zink- und Zinnkomplex-Precursoren in die funktionale Oxidschicht mit halbleitenden Eigenschaften erfolgt in einer weiteren bevorzugten Ausführungsform durch Bestrahlung mit UV-Licht mit Wellenlängen < 400 nm. Bevorzugt liegt die Wellenlänge zwischen 150 und 380 nm. Der Vorteil bei der UV-Bestrahlung liegt darin, dass die dadurch hergestellten Galliumoxid-, Rutheniumoxid-, Magnesiumoxid-, Hafniumoxid-, Indiumzinnoxid- sowie Indium-Gallium-Zinkoxid oder Zink-Zinnoxid-Schichten eine geringere Oberflächenrauhigkeit aufweisen. Eine erhöhte Rauhigkeit der Oberflächen würde nämlich für die dünnen nachfolgenden Schichten eine erhöhte Gefahr bedeuten, dass diese Schichten nicht homogen ausgebildet werden können und damit elektrisch nicht funktionell sind (z.B. Kurzschluss durch eine schadhafte dielektrische Schicht).

Aluminiumoxid-, Galliumoxid-, Neodymoxid, Magnesiumoxid-, Hafniumoxid-oder Zirkoniumoxid-Schichten, die aus den entsprechenden Precursoren hergestellt werden, zeigen zwischen zwei Leitern eine Durchschlagspannung >0.1 MV/cm. Bevorzugt sind Durchschlagspannungen zwischen 1 und 10 MV/cm.
Die Bestimmung der Durchschlagspannung kann mittels der in DIN EN ISO 2376:2009-07 beschriebenen Mess- und Auswerteverfahren erfolgen.

Vorzugsweise besitzt die Indium-Zinnoxid-Schicht (ITO-Schicht) einen spezifischen Widerstand (bestimmt durch Vierpunktmessung) von < 10⁻³ Ohm·cm. Bevorzugt sind spezifische Widerstände zwischen 10⁻³ und 10⁻⁵ Ohm·cm.
Die Leitfähigkeit kann mittels Viersonden-Gleichstromverfahren bestimmt werden. Dieses Messverfahren ist in DIN 50431 oder ASTM F43-99 beschrieben.

Vorzugsweise besitzen die Indium-Gallium-Zinkoxid-Schichten (IGZO) oder die Zink-Zinnoxid- Schichten (ZTO) eine Ladungsträgermobilität > 10⁻³ cm²/Vs. Bevorzugt sind Ladungsträgermobilitäten von 0.1 bis 10 cm²/Vs. Die Charakterisierung und Bestimmung von Kenngrößen halbleitender Materialien kann mittels der in IEEE 1620 beschriebenen Mess- und Auswerteverfahren erfolgen.

Ein weiterer Gegenstand der vorliegenden Erfindung ist die Verwendung eines metallorganischen Aluminium-, Gallium-, Neodym-Ruthenium-, Magnesium-, Hafnium-, Zirkonium-, Indium- und/oder Zinnkomplexes bzw. Precursors zur Herstellung einer oder mehrerer funktioneller Schichten im Feldeffekttransistor.

Das Substrat kann erfindungsgemäß sowohl ein festes Substrat wie Glas, Keramik, Metall oder ein Kunststoffsubstrat als auch ein flexibles Substrat insbesondere Kunststofffolie oder Metallfolie sein. Bevorzugt wird erfindungsgemäß ein flexibles Substrat (Folie) eingesetzt.

Das Aufbringen der erfindungsgemäßen Precursor-Lösungen auf das Substrat durch Verfahren wie Dipcoating, Spincoating und Inkjet-Drucken bzw. Flexo-/Tiefdruck ist dem Fachmann bekannt (siehe M.A. Aegerter, M. Menning; Sol-Gel Technologies for Glass Producers and Users, Kluwer Academic Publishers, Dordrecht, Netherlands, 2004), wobei das Inkjet-Drucken bzw. Flexo-/Tiefdruck erfindungsgemäß bevorzugt ist.

Unter dem Begriff "Feldeffekttransistor (FET)" ist eine Gruppe von unipolaren Transistoren zu verstehen, bei denen im Gegensatz zu den bipolaren Transistoren nur ein Ladungstyp am Stromtransport beteiligt istabhängig von der Bauart Elektronen oder Löcher bzw. Defektelektronen. Die am weitesten verbreitete Art des FET ist der MOSFET (Metall-Oxid-Halbleiter FET)
Der FET verfügt über drei Anschlüsse:
- Source (engl. für "Zufluss", "Quelle")
- Gate (engl. für "Tor", "Gatter")
- Drain (engl. für "Abfluss")

Beim MOSFET ist auch ein vierter Anschluss Bulk (Substrat) vorhanden. Dieser wird bei Einzeltransistoren bereits intern mit dem Source-Anschluss verbunden und nicht extra beschaltet.
Erfindungsgemäß umfasst der Begriff "FET" generell folgende Typen von Feldeffekttransistoren:
- Sperrschicht-Feldeffekt-Transistor (JFET)
- Schottky-Feldeffekt-Transistor (MESFET)
- Metalloxidhalbleiter-FET (MOSFET)
- High Electron Mobility Transistor (HEMT)
- Ionen-Sensitiver Feldeffekt-Transistor (ISFET)
- Dünnschichttransistor (TFT)

Erfindungsgemäß bevorzugt ist der TFT, mit dem großflächige elektronische Schaltungen hergestellt werden können.

Ein weiterer Gegenstand der vorliegenden Erfindung ist ein gedrucktes elektronisches Bauelement welches folgende dünne Schichten aufweist:
- ein festes oder flexibles, leitfähiges Substrat oder ein isolierendes Substrat mit einer leitfähigen Schicht (Gate)
- einen Isolator enthaltend ein Aluminium-, Gallium-,Neodym-, Magnesium-, Hafnium-oder Zirkonium-oxid erhältlich aus dem entsprechenden Precursor des metallorganischen Aluminium-, Gallium-, Neodym-, Magnesium-, Hafnium- oder Zirkoniumkomplexes, der mindestens einen Liganden aus der Klasse der Oximate enthält
- mindestens eine Elektrode (Drain-Elektrode)
- einen Halbleiter.

Die Aluminium-, Gallium-, Neodym-, Magnesium-, Hafnium- oder Zirkonium-oxid-Schicht hat eine Dicke von 15 nm bis 1 µm, vorzugsweise 30 nm bis 750 nm. Die Schichtdicke ist abhängig von der jeweils verwendeten Beschichtungstechnik und deren Parametern. Im Falle von Spincoating sind dies beispielsweise die Rotationsgeschwindigkeit und - dauer.

Ein weiterer Gegenstand der vorliegenden Erfindung ist ein gedrucktes elektronisches Bauelement welches folgende dünne Schichten aufweist:
- ein festes oder flexibles, leitfähiges Substrat oder ein isolierendes Substrat mit einer leitfähigen Schicht (Gate) enthaltend ein Indium-Zinn-oxid (ITO) erhältlich aus den entsprechenden Precursoren der metallorganischen Indium- und Zinnkomplexe, die mindestens einen Liganden aus der Klasse der Oximate enthalten,
- einen Isolator
- mindestens eine Elektrode (Drain-Elektrode)
- einen Halbleiter.

Die Indium-Zinn-Oxid-Schicht (ITO-Schicht) hat eine Dicke von 15 nm bis 1 µm, vorzugsweise 100 nm bis 500 nm. Die Schichtdicke ist abhängig von der jeweils verwendeten Beschichtungstechnik und deren Parametern. Im Falle von Spincoating sind dies beispielsweise die Rotationsgeschwindigkeit und -dauer.

Ein weiterer Gegenstand der vorliegenden Erfindung ist ein gedrucktes elektronisches Bauelement welches folgende dünne Schichten aufweist:
- ein festes oder flexibles, leitfähiges Substrat oder ein isolierendes Substrat mit einer leitfähigen Schicht (Gate)
- einen Isolator
- mindestens eine Elektrode (Drain-Elektrode)
- einen Halbleiter. Der Halbleiter soll aus einer multinären, amorphen Phase aus Indium-Gallium-Zinkoxid (IGZO) oder aber aus Zink-Zinnoxid (ZTO) bestehen, erhältlich aus den entsprechenden Precursoren der metallorganischen Indium-, Gallium-, Zinkkomplexe bzw. der Zink- und Zinnkomplexe, die mindestens einen Liganden aus der Klasse der Oximate enthalten.

Die Indium-Gallium-Zinkoxid (IGZO)- oder Zink-Zinnoxid (ZTO) -Schicht hat eine Dicke von 15 nm bis 1 µm, vorzugsweise 20 nm bis 200 nm. Die Schichtdicke ist abhängig von der jeweils verwendeten Beschichtungstechnik und deren Parametern. Im Falle von Spincoating sind dies beispielsweise die Rotationsgeschwindigkeit und -dauer.

In einer bevorzugten Ausführungsform bestehen die zuvor genannten elektronischen Bauelemente aus einem Feldeffekttransistor bzw. Dünnschichttransistor, welcher aus einem Gate, einer isolierenden Schicht, einem Halbleiter und Elektroden (Drain und Source) aufgebaut ist. Bevorzugt besteht das Gate aus einem hoch-n-dotierten Silicium-Wafer, einer hoch-n-dotierten Siliciumdünnschicht, leitfähigen Polymeren (z.B. Polypyrrol-Polyaminobenzolsulfonsäure oder Polyethylendioxythiophen-Polystyrolsulfonsäure (PEDOT-PSS)), leitfähigen Keramiken (z.B. Indium-Zinn-Oxid (ITO) oder Al, Ga oder In-dotiertes Zinnoxid (AZO, GZO, IZO) sowie F oder Sb dotiertes Zinnoxid (FTO, ATO)) oder Metallen (z.B. Gold, Silber, Titan, Zink), je nach Ausführung als dünne Schicht oder Substratmaterial. Die dünnen Schichten können je nach Ausführung in der Anordnung unterhalb (Bottom-Gate) oder oberhalb (Top-Gate) der halbleitenden beziehungsweise der isolierenden Schicht aufgebracht sein. Bevorzugt besitzt das elektronische Bauelement eine isolierende Schicht die aus Polymeren (z.B. Poly(4-vinylphenol), Polymethylmethacrylat, Polystyrol, Polyimiden oder Polycarbonat) oder Keramiken (z.B. Siliziumdioxid, Siliziumnitrid, Aluminiumoxid, Galliumoxid, Neodymoxid, Magnesiumoxid, Hafniumoxid, Zirkoniumoxid) besteht.

Bevorzugt besitzt das elektronische Bauelement eine halbleitende Schicht die aus einer halbleitenden organischen Verbindung (z.B. Polythiophen, Oligothiophen, oder Polytriarylamin) oder Keramiken (z.B. Zinkoxid, Indium-Gallium-Zinkoxid (IGZO) oder Zink-Zinnoxid (ZTO)) besteht.

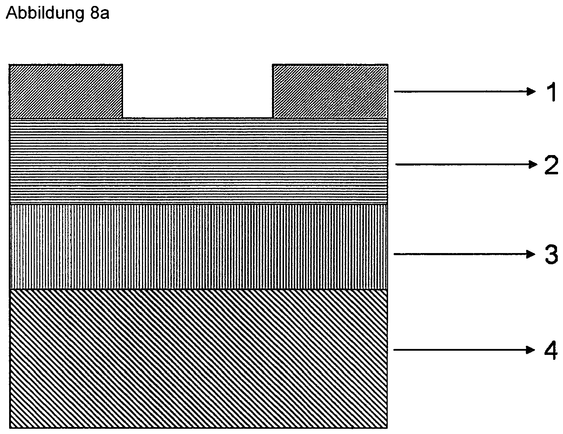

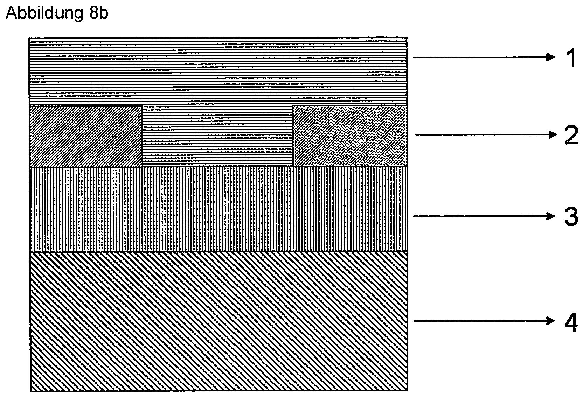

Bevorzugt besitzt das elektronische Bauteil Source- und Drain-Elektroden aus einer hoch-n-dotierten Siliciumdünnschicht, leitfähigen Polymeren (z.B. Polypyrrol-Polyaminobenzolsulfonsäure oder Polyethylendioxythiophen-Polystyrolsulfonsäure (PEDOT-PSS)), leitfähigen Keramiken (z.B. Indium-Zinn-Oxid (ITO) oder Al, Ga oder In-dotiertes Zinnoxid (AZO, GZO, IZO) sowie F oder Sb dotiertes Zinnoxid (FTO, ATO)) oder Metallen (z.B. Gold, Silber, Titan, Zink). Die Elektroden (erfindungsgemäß bevorzugt als dünne Schichten ausgeführt) können je nach Ausführung in der Anordnung unterhalb (Bottom-Contact) oder oberhalb (Top-Contact) der halbleitenden beziehungsweise der isolierenden Schicht aufgebracht sein (siehe Abbildung 8a und b).

In der oben genannten bevorzugten Ausführungsform können Gate, Isolator und Halbleiter unstrukturiert mittels Spincoating oder Dipcoating, sowie Abscheidungstechniken aus der gasförmigen oder flüssigen Phase aufgebracht werden. Weiter können Gate, Isolator, Halbleiter und Elektroden strukturiert mittels Flexo-/Tiefdruck, Inkjetdruck sowie Abscheidungstechniken aus der gasförmigen oder flüssigen Phase aufgebracht werden. Bevorzugt sind erfindungsgemäss Druckverfahren.

Ein weiterer Gegenstand der vorliegenden Erfindung ist ein Verfahren zur Herstellung von elektronischen Strukturen mit Aluminium-, Gallium-, Neodym-, Ruthenium-, Magnesium-, Hafnium-, Zirkonium-, Indium- und/oder Zinn-oxid-Schicht oder -Oberfläche sowie deren Gemische, gekennzeichnet dadurch, dass
a) Precursor-Lösungen aus einem metallorganischen Aluminium-, Gallium-, Neodym-, Ruthenium-, Magnesium-, Hafnium-, Zirkonium-, Indium- und/oder Zinnkomplex sowie deren Gemische, die mindestens einen Liganden aus der Klasse der Oximate enthalten, schichtartig wahlweise ein- oder mehrfach entsprechend der zu erzielenden elektronischen Struktur durch Dipcoating, Spincoating oder Inkjet-Drucken bzw. Flexo/Tiefdruck auf ein Substrat aufgebracht werden,
b) Tempern oder Trocknen der aufgebrachten Precursor-Schicht an Luft oder Sauerstoff-Atmosphäre unter Bildung einer Aluminium-, Gallium-, Neodym-, Ruthenium-, Magnesium-, Hafnium-, Zirkonium-, Indium-, Zinn- oder Indiumzinnoxid-Schicht oder Oberfläche sowie deren Gemische,
c) abschließend die aufgebrachte elektronische Struktur mit einer isolierenden Schicht versiegelt werden kann sowie kontaktiert und komplettiert wird.

Durch dieses Verfahren werden sowohl elektronische Bauelemente als auch die Verbindungen einzelner Bauelemente in integrierten Schaltungen hergestellt.

Die folgenden Beispiele sollen die vorliegende Erfindung verdeutlichen. Sie sind jedoch keinesfalls als limitierend zu betrachten. Alle Verbindungen oder Komponenten, die in den Zubereitungen verwendet werden können, sind entweder bekannt und käuflich erhältlich oder können nach bekannten Methoden synthetisiert werden.

### Beispiel 1: Herstellung des Magnesiumoxid-Precursors Bis[2-(Methoxyimino)propanoato]-Magnesium

Zu einer Lösung von Natriumpyruvat (6.60 g, 60 mmol) und Methoxylaminhydrochlorid (5.01 g, 60 mmol) in 100 mL Wasser wird unter Rühren Kaliumhydrogencarbonat (12.02 g, 120 mmol) in kleinen Portionen gegeben. Nach Beendigung der sichtbaren Gasentwicklung wird noch für weitere 30 Minuten gerührt. Anschliessend wird Magnesiumnitrat-Hexahydrat (7.69 g, 30 mmol) zugegeben und noch eine Stunde gerührt. Die klare Lösung wird am Rotationsverdampfer auf die Hälfte des Volumens eingeengt und auf etwa 5 Grad abgekühlt. Der weisse Niederschlag, der sich gebildet hat, wird abfiltriert und aus heissem Wasser umkristallisiert. Ausbeute 2.60 g (34.8%) Die so erhaltene Verbindung kann durch IR- und NMR-Spektroskopie charakterisiert werden.

### Beispiel 2: Alkalimetallfreie Herstellung des Magnesiumoxid-Precursors Bis[2-(Methoxyimino)propanoato]-Magnesium

Alternativ dazu ist nachfolgende Reaktionsführung möglich.
Zu einer Lösung von Brenztraubensäure (10.56 g, 120mmol) und Methoxylaminhydrochlorid (10.04 g, 120 mmol) in 100 mL Wasser wird Hydromagnesit (20.0 g) portionsweise zugefügt. Nach Beendigung der sichtbaren Gasentwicklung wird für eine Stunde gerührt und nicht umgesetztes Hydromagnesit abfiltriert. Die klare Lösung wird am Rotationsverdampfer auf die Hälfte des Volumes eingeengt und auf etwa 5 Grad abgekühlt. Der weisse Niederschlag, der sich gebildet hat, wird abfiltriert und aus heissem Wasser umkristallisiert. Ausbeute 6.50 g (43.5%) Die so erhaltene Verbindung kann durch IR- und NMR-Spektroskopie charakterisiert werden.

### Beispiel 3: Herstellung von undotierten Magnesiumoxid-Schichten aus dem Magnesium-Precursor mit Isolator-Eigenschaften (aus Beispiel 1 oder 2)

Das nach Beispiel 1 oder 2 hergestellte Magnesiumoximat wird mittels Spincoating (oder Dipcoating oder auch Inkjet-Drucken) auf ein Substrat aus Glas, Keramik oder Polymer aufgebracht. Anschließend wird an der Luft bei Temperaturen über 210°C für 10 Minuten getempert. Die so erhaltenen Magnesiumoxid-Filme zeigen eine gleichförmige, rissfreie, nicht-poröse Oberflächenmorphologie. In Abhängigkeit von der Calcinierungstemperatur bestehen die Schichten aus amorphen oder nanokristallinem Material. Die Schichten besitzen Isolator-Eigenschaften.

### Beispiel 4: Herstellung eines Zirkoniumdioxid-Precursors Zirkoniumhydroxo[2-(Methoxyimino)propanoat], Vergleichsbeispiel

Zu einer Lösung von Natriumpyruvat (9.90 g, 90 mmol) und Methoxylaminhydrochlorid (7.53 g, 90 mmol) 100 mL Wasser wird in kleinen Portionen unter Rühren Natriumhydrogencarbonat (7.56 g, 90 mmol) gegeben. Nach Beendigung der sichtbaren Gasentwicklung wird noch für 30 Minuten gerührt. Anschließend wird am Rotationsverdampfer bis zur völligen Trockene eingeengt. Zu dem so erhaltenen weissen Pulver wird eine Lösung von Zirkoniumtetrachlorid (5.24 g, 22.5 mmol) in 125 mL Tetrahydrofuran gegeben und für zwei Stunden gerührt. Die Lösung wird abfiltriert und am Rotationsverdampfer bis zur Trockene eingeengt. Der Rückstand wird in 100 mL Dichlormethan aufgenommen und die so erhaltene Suspension erneut filtriert. Anschließend wird das Produkt aus dem Filtrat mit reichlich n-Hexan gefällt, abfiltriert und im Exsikkator getrocknet. Ausbeute 3,50 g. Die so erhaltene Verbindung kann durch IR-und NMR-Spektroskopie charakterisiert werden.

### Beispiel 5: Herstellung von Zirkoniumoxid-Schichten aus dem Zirkoniumdioxid-Precursor

Das nach Beispiel 4 hergestellte Zirkoniumoximat wird mittels Spincoating (oder Dipcoating oder auch Inkjet-Drucken) auf ein Substrat aus Glas, Keramik oder Polymer aufgebracht. Anschliessend wird an der Luft bei Temperaturen über 200°C für 10 Minuten getempert oder UV-Bestrahlung mit 150 mW/cm² für 15 Minuten durchgeführt. Die so erhaltenen Zirkoniumoxid-Filme zeigen eine gleichförmige, rissfreie, nicht-poröse Oberflächenmorphologie. In Abhängigkeit von der Calcinierungstemperatur bestehen die Schichten aus amorphen oder nanokristallinem Material. Die Schichten besitzen Isolator-Eigenschaften.

### Beispiel 6: Herstellung des Hafniumdioxid-Precursors Hafniumhydroxo[2-(Methoxyimino)propanoat]

Zu einer Lösung von Natriumpyruvat (9.90 g, 90 mmol) und Methoxylaminhydrochlorid (7.53 g, 90 mmol) 100 mL Wasser wird in kleinen Portionen unter Rühren Natriumhydrogencarbonat (7.56 g, 90 mmol) gegeben. Nach Beendingung der sichtbaren Gasentwicklung wird noch für 30 Minuten gerührt. Anschliessend wird am Rotationsverdampfer bis zur völligen Trockene eingeengt. Zu dem so erhaltenen weissen Pulver wird eine Lösung von Hafniumtetrachlorid (7.21 g, 22.5 mmol) in 125 mL Tetrahydrofuran gegeben und für zwei Stunden gerührt. Die Lösung wird abfiltiert und am Rotationsverdampfer bis zur Trockene eingeengt. Der Rückstand wird in 100 mL Dichlormethan aufgenommen und die so erhaltene Suspension erneut filtriert. Anschliessend wird das Produkt aus dem Filtrat mit reichlich n-Hexan gefällt, abfiltriert und im Exsikkator getrocknet. Ausbeute 4.75 g. Die so erhaltene Verbindung kann durch IR-und NMR-Spektroskopie charakterisiert werden.

### Beispiel 7: Herstellung von Hafniumoxid-Schichten mit Isolator-Eigenschaften

Das nach Beispiel 6 hergestellte Hafniumoximat wird mittels Spincoating (oder Dipcoating oder auch Inkjet-Drucken) auf ein Substrat aus Glas, Keramik oder Polymer aufgebracht. Anschliessend wird an der Luft bei Temperaturen über 200°C für 10 Minuten getempert oder UV-Bestrahlung mit 150 mW/cm² für 15 Minuten durchgeführt. Die so erhaltenen Hafniumoxid-Filme zeigen eine gleichförmige, rissfreie, nicht-poröse Oberflächenmorphologie. In Abhängigkeit von der Calcinierungstemperatur bestehen die Schichten aus amorphem oder nanokristallinem Material. Die Schichten besitzen Isolator-Eigenschaften.

### Beispiel 8: Herstellung eines Aluminiumoxid-Precursors Tris[2-(Methoxyimino)propanoato]-Aluminium, Vergleichsbeispiel

Zu einer Lösung von Natriumpyruvat (2.20 g, 20 mmol) und Methoxylaminhydrochlorid (1.67 g, 20 mmol) 50 mL Wasser wird in kleinen Portionen unter Rühren Natriumhydrogencarbonat (1.68 g, 20 mmol) gegeben. Nach Beendigung der sichtbaren Gasentwicklung wird noch für 30 Minuten gerührt. Anschliessend wird am Rotationsverdampfer bis zur völligen Trockene eingeengt. Zu dem so erhaltenen weissen Pulver wird eine Lösung von Aluminiumnitratnonahydrat (2.50 g, 6.6 mmol) in 125 mL Methanol gegeben und für zwei Stunden gerührt. Die Lösung wird abfiltiert und am Rotationsverdampfer bis zur Trockene eingeengt. Der Rückstand wird in 50 mL Dichlormethan aufgenommen und die so erhaltene Suspension erneut filtriert. Anschliessend wird das Produkt aus dem Filtrat mit reichlich n-Hexan gefällt, abfiltriert und im Exsikkator getrocknet. Ausbeute 1.12 g (45.30 %). Die so erhaltene Verbindung kann durch IR-und NMR-Spektroskopie charakterisiert werden.

### Beispiel 9: Herstellung von Aluminiumoxid-Schichten mit Isolator-Eigenschaften

Das nach Beispiel 8 hergestellte Aluminiumoximat wird mittels Spincoating (oder Dipcoating oder auch Inkjet-Drucken) auf ein Substrat aus Glas, Keramik oder Polymer aufgebracht. Anschliessend wird an der Luft bei Temperaturen über 200°C für 10 Minuten getempert oder UV-Bestrahlung mit 150 mW/cm² für 15 Minuten durchgeführt. Die so erhaltenen Aluminiumoxid-Filme zeigen eine gleichförmige, rissfreie, nicht-poröse Oberflächenmorphologie. In Abhängigkeit von der Calcinierungstemperatur bestehen die Schichten aus nanokristallinem Material. Die Schichten besitzen Isolator-Eigenschaften.

### Beispiel 10: Herstellung des Galliumoxid-Precursors Tris[2-(Methoxyimino)propanoato]-Gallium

Zu einer Lösung von Natriumpyruvat (2.20 g, 20 mmol) und Methoxylaminhydrochlorid (1.67 g, 20 mmol) 50 mL Wasser wird in kleinen Portionen unter Rühren Natriumhydrogencarbonat (1.68 g, 20 mmol) gegeben. Nach Beendigung der sichtbaren Gasentwicklung wird noch für 30 Minuten gerührt. Anschliessend wird am Rotationsverdampfer bis zur völligen Trockene eingeengt. Zu dem so erhaltenen weissen Pulver wird eine Lösung von Galliumnitrathexahydrat (2.40 g, 6.6 mmol) in 125 mL Methanol gegeben und für zwei Stunden gerührt. Die Lösung wird abfiltiert und am Rotationsverdampfer bis zur Trockene eingeengt. Der Rückstand wird in 100 mL Dichlormethan aufgenommen und die so erhaltene Suspension erneut filtriert. Anschliessend wird das Produkt aus dem Filtrat mit reichlich n-Hexan gefällt, abfiltriert und im Exsikkator getrocknet. Ausbeute 0.96 g (34.82 %). Die so erhaltene Verbindung kann durch IR-und NMR-Spektroskopie charakterisiert werden.

### Beispiel 11: Herstellung von Galliumoxid-Schichten mit Isolator-Eigenschaften

Das nach Beispiel 10 hergestellte Galliumoximat wird mittels Spincoating (oder Dipcoating oder auch Inkjet-Drucken) auf ein Substrat aus Glas, Keramik oder Polymer aufgebracht. Anschliessend wird an der Luft bei Temperaturen über 200°C für 10 Minuten getempert oder UV-Bestrahlung mit 150 mW/cm² für 15 Minuten durchgeführt. Die so erhaltenen Galliumoxid-Filme zeigen eine gleichförmige, rissfreie, nicht-poröse Oberflächenmorphologie. In Abhängigkeit von der Calcinierungstemperatur bestehen die Schichten aus nanokristallinem Material. Die Schichten besitzen Isolator-Eigenschaften.

### Beispiel 12: Herstellung eines Neodymoxid-Precursors Tris[2-(Methoxyimino)propanoato]-Neodym, Vergleichsbeispiel

Zu einer Lösung von Natriumpyruvat (2.20 g, 20 mmol) und Methoxylaminhydrochlorid (1.67 g, 20 mmol) 50 mL Wasser wird in kleinen Portionen unter Rühren Natriumhydrogencarbonat (1.68 g, 20 mmol) gegeben. Nach Beendigung der sichtbaren Gasentwicklung wird noch für 30 Minuten gerührt. Anschliessend wird am Rotationsverdampfer bis zur völligen Trockene eingeengt. Zu dem so erhaltenen weissen Pulver wird eine Lösung von wasserfreiem Neodymtrichlorid (1.65g, 6.6 mmol) in 125 mL Methanol gegeben und für zwei Stunden gerührt. Die Lösung wird abfiltiert und am Rotationsverdampfer bis zur Trockene eingeengt. Der Rückstand wird in 100 mL Dichlormethan aufgenommen und die so erhaltene Suspension erneut filtriert. Anschliessend wird das Produkt aus dem Filtrat mit reichlich n-Hexan gefällt, abfiltriert und im Exsikkator getrocknet. Ausbeute 1.55 g (47.83 %). Die so erhaltene Verbindung kann durch IR- und NMR-Spektroskopie charakterisiert werden.

### Beispiel 13: Herstellung von Neodymoxid-Schichten mit Isolator-Eigenschaften

Das nach Beispiel 12 hergestellte Neodymoximat wird mittels Spincoating (oder Dipcoating oder auch Inkjet-Drucken) auf ein Substrat aus Glas, Keramik oder Polymer aufgebracht. Anschliessend wird an der Luft bei Temperaturen über 200°C für 10 Minuten getempert oder UV-Bestrahlung mit 150 mW/cm² für 15 Minuten durchgeführt. Die so erhaltenen Neodymoxid-Filme zeigen eine gleichförmige, rissfreie, nicht-poröse Oberflächenmorphologie. In Abhängigkeit von der Calcinierungstemperatur bestehen die Schichten aus nanokristallinem Material. Die Schichten besitzen Isolator-Eigenschaften.

### Beispiel 14: Herstellung des Rutheniumoxid-Precursors Tris[2-(Methoxyimino)propanoato]-Ruthenium

Zu einer Lösung von Natriumpyruvat (2.20 g, 20 mmol) und Methoxylaminhydrochlorid (1.67 g, 20 mmol) 50 mL Wasser wird in kleinen Portionen unter Rühren Natriumhydrogencarbonat (1.68 g, 20 mmol) gegeben. Nach Beendigung der sichtbaren Gasentwicklung wird noch für 30 Minuten gerührt. Anschliessend wird am Rotationsverdampfer bis zur völligen Trockene eingeengt. Zu dem so erhaltenen weissen Pulver wird eine Lösung von Rutheniumtrichloridhexahydrat (2.08g, 6.6 mmol) in 125 mL Methanol gegeben und für zwei Stunden gerührt. Die Lösung wird abfiltiert und am Rotationsverdampfer bis zur Trockene eingeengt. Der Rückstand wird in 100 mL Dichlormethan aufgenommen und die so erhaltene Suspension erneut filtriert. Anschliessend wird das Produkt aus dem Filtrat mit reichlich Toluol gefällt, abfiltriert und im Exsikkator getrocknet. Ausbeute 1.34 g (45.33 %). Die so erhaltene Verbindung kann durch IR- und NMR-Spektroskopie charakterisiert werden.

### Beispiel 15: Herstellung von Rutheniumoxid-Schichten

Das nach Beispiel 14 hergestellte Rutheniumoximat wird mittels Spincoating (oder Dipcoating oder auch Inkjet-Drucken) auf ein Substrat aus Glas, Keramik oder Polymer aufgebracht. Anschliessend wird an der Luft bei Temperaturen über 200°C für 10 Minuten getempert oder UV-Bestrahlung mit 150 mW/cm² für 15 Minuten durchgeführt. Die so erhaltenen Rutheniumoxid-Filme zeigen eine gleichförmige, rissfreie, nicht-poröse Oberflächenmorphologie. In Abhängigkeit von der Calcinierungstemperatur bestehen die Schichten aus nanokristallinem Material.

### Beispiel 16: Darstellung von dielektrischen Schichten

Das Beispiel zeigt die Herstellung einer dielektrischen Schicht. Auf einen leitenden hoch-n-dotierten Silicium-Wafer wird eine Schicht von Zirkoniumoxid durch Spincoaten oder Drucken aufgebracht und durch anschließendes Calcinieren bei 200°C für 10 Minuten oder UV Bestrahlung mit 150 mW/cm² für 15 Minuten dargestellt. Nach Aufbringen einer Gegenelektrode kann der Wert für die Durchschlagspannung für die dielektrische Schicht mit 1 MV/cm ermittelt werden.

### Beispiel 17: Herstellung von Dünnschichttransistoren

Das Beispiel zeigt einen Feldeffekttransistor (FET) gemäß Abbildungen 8a und 8b. Das Bauelement besteht aus einem hoch-n-dotierten Silicium-Wafer, auf den die dielektrische Schicht aufgebracht ist. Sodann wird eine halbleitende Schicht dargestellt, gefolgt von einer Elektrodenstruktur aus Metall die durch Aufdampfen des Metalls oder Drucken einer Metalltinte hergestellt wird (Abbildung 8a). Alternativ dazu kann zunächst die Herstellung der Elektrodenstruktur und danach das Aufbringen der Halbleiterschicht erfolgen (Abbildung 8b).

### Beispiel 18: Herstellung des Indiumoxid-Precursors Tris[2-(Methoxyimino)propanoato]-Indium

Zu einer Lösung von Natriumpyruvat (2.20 g, 20 mmol) und Methoxylaminhydrochlorid (1.67 g, 20 mmol) 50 mL Wasser wird in kleinen Portionen unter Rühren Natriumhydrogencarbonat (1.68 g, 20 mmol) gegeben. Nach Beendigung der sichtbaren Gasentwicklung wird noch für 30 Minuten gerührt. Anschließend wird am Rotationsverdampfer bis zur völligen Trockene eingeengt. Zu dem so erhaltenen weißen Pulver wird eine Lösung von wasserfreiem Indiumchlorid (1.95 g, 6.6 mmol) in 125 mL Methanol gegeben und für zwei Stunden gerührt. Die Lösung wird abfiltiert und am Rotationsverdampfer bis zur Trockene eingeengt. Der Rückstand wird in 100 mL Dichlormethan aufgenommen und die so erhaltene Suspension erneut filtriert. Anschließend wird das Produkt aus dem Filtrat mit reichlich n-Hexan gefällt, abfiltriert und im Exsikkator getrocknet. Ausbeute 1.80 g (58.9 %). Die so erhaltene Verbindung kann durch IR- und NMR-Spektroskopie charakterisiert werden.

### Beispiel 19: Herstellung des Zinnoxid-Precursors Zinnhydroxo[2-(Methoxyimino)propanoat]

Zu einer Lösung von Natriumpyruvat (9.90 g, 90 mmol) und Methoxylaminhydrochlorid (7.53 g, 90 mmol) 100 mL Wasser wird in kleinen Portionen unter Rühren Natriumhydrogencarbonat (7.56 g, 90 mmol) gegeben. Nach Beendigung der sichtbaren Gasentwicklung wird noch für 30 Minuten gerührt. Anschließend wird am Rotationsverdampfer bis zur völligen Trockene eingeengt. Zu dem so erhaltenen weißen Pulver wird eine Lösung von wasserfreiem Zinnchlorid-Pentahydrat (7.88 g. 22.5 mmol) in 250 mL Methanol gegeben und für zwei Stunden gerührt. Die Lösung wird abfiltiert und am Rotationsverdampfer bis zur Trockene eingeengt. Der Rückstand wird in 100 mL Aceton oder Dimethoxyethan aufgenommen und die so erhaltene Suspension erneut filtriert. Anschließend wird das Produkt aus dem Filtrat mit reichlich Diethlyether gefällt, abfiltriert und im Exsikkator getrocknet. Ausbeute 3.7 g. Die so erhaltene Verbindung kann durch IR- und NMR-Spektroskopie charakterisiert werden.

### Beispiel 20: Herstellung elektrisch leitfähiger Schichten von Indium-Zinn-Oxid (ITO)

Die nach Beispiel 18 und 19 hergestellte Indium- und Zinnoximate werden zusammen im molaren Verhältnis 90:10 in Lösung gebracht und mittels Spincoating (oder Dipcoating oder auch Inkjet-Drucken) auf ein Substrat aus Glas, Keramik oder Polymer aufgebracht. Anschließend wird an der Luft bei Temperaturen über 200°C für 60 Minuten getempert oder UV-Bestrahlung mit 150 mW/cm² für 1 Stunde durchgeführt. Die so erhaltenen Indium-Zinnoxid-Filme zeigen eine gleichförmige, rissfreie, nicht-poröse Oberflächenmorphologie. In Abhängigkeit von der Calcinierungstemperatur bestehen die Schichten aus amorphen oder nanokristallinem Material. Die Schichten besitzen elektrische Leitfähigkeit.

### Beispiel 21: Herstellung halbleitender Schichten aus Indium-Gallium-Zinkoxid (IGZO)

Die nach Beispiel 10 und 18 hergestellten Indium- und/oder Galliumoximate, sowie Zinkoximate (zugänglich nach J.J. Schneider et al. Advanced Materials, 2008, 20, 3383-3387) werden in einem geeigneten molaren Verhältnis in Lösung gebracht. Geeignete molare Verhältnisse zur Herstellung halbleitender Phasen finden sich in H. Hosono, Journal of Non-Crystalline Solids 2006, 352, 851-858; so z.B. Indium: Gallium: Zink = 1 :1 :1 oder Indium:Zink = 2:3.

Die so erhaltene Lösung wird mittels Spincoating (oder Dipcoating oder auch Inkjet-Drucken) auf ein Substrat aus Glas, Keramik oder Polymer aufgebracht. Anschließend wird an der Luft bei Temperaturen über 200°C für 10 Minuten getempert oder UV-Bestrahlung mit 150 mW/cm² für 15 Minuten durchgeführt. Die so erhaltenen Indium-Gallium-Zinkoxid -Filme zeigen eine gleichförmige, rissfreie, nicht-poröse Oberflächenmorphologie. In Abhängigkeit von der Calcinierungstemperatur bestehen die Schichten aus amorphen Material. Die Schichten besitzen halbleitende Eigenschaften.

### Beispiel 22: Herstellung halbleitender Schichten aus Zink-Zinnoxid (ZTO)

Die nach Beispiel 19 hergestellten Zinnoximate sowie Zinkoximate (zugänglich nach J.J. Schneider et al. Advanced Materials, 2008, 20,3383-3387) werden zusammen in einem geeigneten Verhältnis in Lösung gebracht. Geeignet sind z.B. Zinngehalte Sn/(Sn+Zn) von 0 bis 30 Molprozent. Die Lösung wird mittels Spincoating (oder Dipcoating, oder auch Inkjet-Drucken) auf ein Substrat aus Glas, Keramik oder Polymer aufgebracht. Anschließend wird an der Luft bei Temperaturen über 200°C für 10 Minuten getempert oder UV-Bestrahlung mit 150 mW/cm² für 15 Minuten durchgeführt. Die so erhaltenen Zink-Zinnoxid -Filme zeigen eine gleichförmige, rissfreie, nicht-poröse Oberflächenmorphologie. In Abhängigkeit von der Calcinierungstemperatur bestehen die Schichten aus amorphen oder nanokristallinem Material. Die Schichten besitzen halbleitende Eigenschaften.

### Beispiele 23 bis 26: Beschreibung verschiedener Coatingprozesse

In allen Fällen werden Lösungen von 10-20 Gewichtsprozent der Precursor-Verbindungen verwendet. Als Lösemittel eignen sich 2-Methoxyethanol, 2-Butanol, Methanol, Dimethylformamid oder Gemische daraus.
Die Viskosität der Drucktinten kann mit einem Rheometer, z.B. MARS Rheometer der Fa. Haake, bestimmt werden. Die Bestimmung erfolgt unter Normklima (DIN 50 014-23).

**Dipcoating:** Zuggeschwindigkeit ∼1mm/sec. Als Substrate werden Glasplättchen 76x26 mm eingesetzt.
**Spincoating:** Für das Spincoating werden 150 µL Lösung auf das Substrat aufgetragen. Als Substrate wird Quarz 20x20 mm oder Silicium (mit Goldelektroden zur Herstellung der FET) 15x15 mm verwendet. Als Parameter für Dauer und Geschwindigkeit werden 10s bei einer Vorlaufgeschwindigkeit von 1000 rpm sowie 20 s bei der Endgeschwindigkeit von 2000 rpm gewählt.
**Inkjet-Druck:** Der Druckprozess kann mit dem Drucker Dimatix DMP 2831 der Firma Dimatix durchgeführt werden. Als Drucktinte wird eine Lösung von 10 Gewichtsprozent des Hafniumoxidprecursors Hafniumhydroxo[2-(Methoxyimino)propanoat] in Diethylenglycolmonoethylether verwendet, die unter Verwendung eines Spritzenfilter mit 0,2 µm Porengröße eingefüllt wird. Filme können auf Substraten wie Glass mit Beschichtung aus Indiumzinnoxid (Fa. Merck) gedruckt und durch anschließende UV Bestrahlung (Fe dotierte Hg Lampe; Bestrahlungsdauer: 4 min bei 400 mW/cm²) in die Keramik umgewandelt werden.
**Flexo-Druck:** Der Druckprozess kann mit dem Gerät IGT F1 der Fa. IGT Testing Systems durchgeführt werden.
Als Drucktinte wird eine Lösung von 10 Gewichtsprozent des Hafniumoxidprecursors Hafniumhydroxo[2-(Methoxyimino)propanoat] in Methoxyethanol verwendet. Die Auftragung von 1 ml Lösung in der Kontaktzone erfolgt manuell.
Für Rakelblech/Rasterwalze werden folgende Druckparameter gewählt:
- Anstellkraft Rasterwalze/Formzylinder 10 N
- Anstellkraft Formzylinder/Gegendruckzylinder 10 N
- Druckgeschwindigkeit 0,8m/s
- Druckform: Volltonfläche, 90% Flächendeckung
- Rasterwalze Schöpfvolumen 20 ml/m²
Vollflächige Bedruckung kann auf Substraten wie Silicium oder Quarz erzielt werden. Nachfolgend kann der Precursor in die Keramik durch UV-Bestrahlung (Fe dotierte Hg Lampe; Bestrahlungsdauer: 7 min bei 400 mW/cm²) umgewandelt werden.

### Verzeichnis der Abbildungen

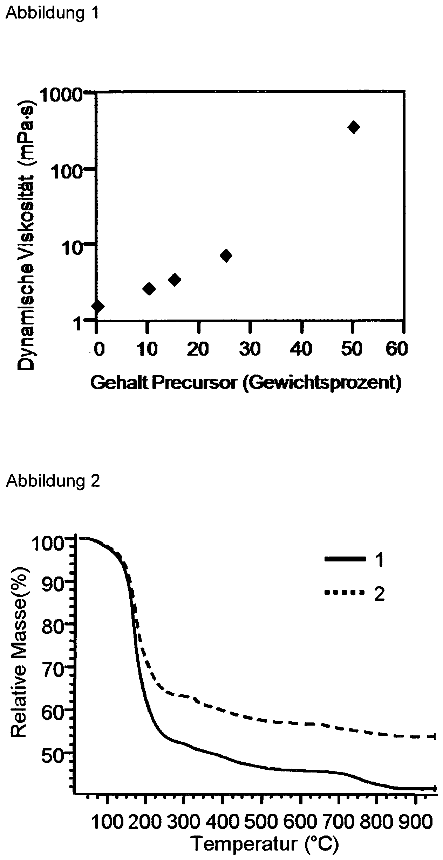

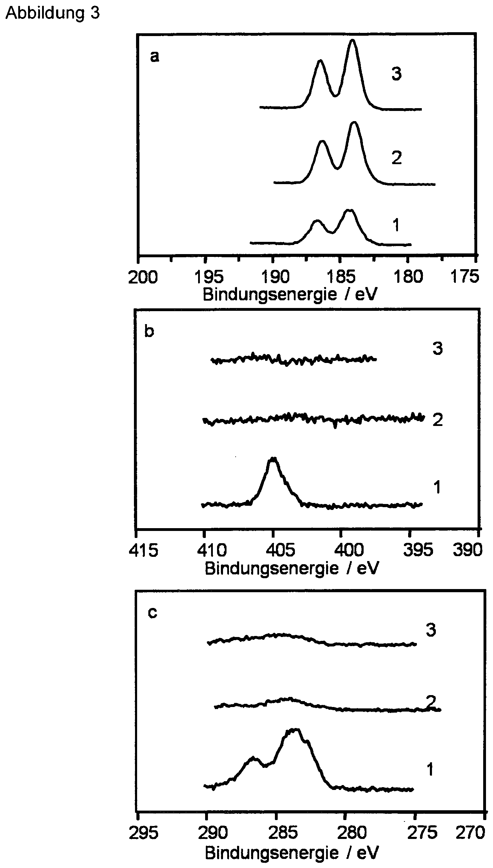

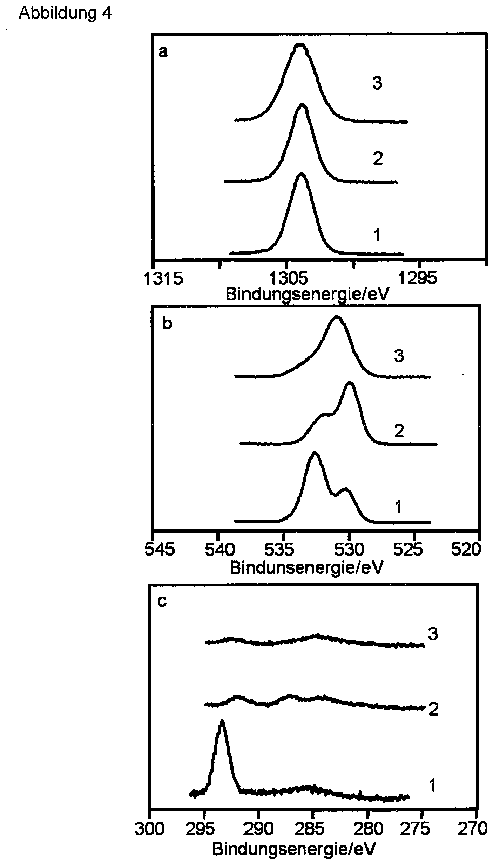

Im Folgenden soll die Erfindung anhand mehrerer Ausführungsbeispiele (siehe Abbildungen 1 bis 8b) näher erläutert werden.
Abb. 1: zeigt die Abhängigkeit der dynamischen Viskosität vom Precursorgehalt für Lösungen von Hafniumhydroxo[2-(Methoxyimino)propanoat] in Methoxyethanol. Die Messung erfolgte unter Normklima (DIN 50 014-23).
Abb. 2: zeigt eine Thermogravimetrische Analyse des Zirkonium- sowie des Hafniumhydroxo[2-(Methoxyimino)propanoat] in Sauerstoff (1= Kurve für Zirkoniumkomplex, 2 = Kurve für Hafniumkomplex). Die Abnahme der relativen Masse in Abhängigkeit von der Temperatur erlaubt eine Aussage über die erforderlichen Umsetzungstemperaturen.
Abb.3: zeigt eine Analyse mittels Röntgenphotonenspektroskopie (XPS) der erfindungsgemäßen Filme aus Zirkonium-Oximat in 2-Methoxyethanol/2-Butanol durch Spincoating auf Siliciumsubstraten und Prozessierung bei verschiedenen Temperaturen für jeweils 30 Minuten. (a=Zr3d-Signal. b= N1s-Signal. c=C1s-Signal. 1=ohne weitere Prozessierung. 2=Prozessierung bei 200°C. 3=Prozessierung bei 250°C.)
   Die XPS-Spektren erlauben eine Aussage über die in der Probe vorhandenen Elemente und der Oxidationsstufe, sowie über das Mengenverhältnis. Damit kann gezeigt werden, dass in den Filmen nach der Prozessierung Zirkoniumoxid vorliegt.
Abb. 4: zeigt eine Analyse mittels Röntgenphotonenspektroskopie (XPS) der erfindungsgemäßen Filme aus Magnesium-Oximat in 2-Methoxyethanol durch Spincoating auf Siliciumsubstraten und Prozessierung bei verschiedenen Temperaturen für jeweils 30 Minuten.
   Bei einigen Messungen wurde die Oberfläche in der Messkammer durch Sputtern gereinigt. (a=Mg1s-Signal. b= 01s-Signal. c=C1s-Signat. 1=Prozessierung bei 210°C+Sputtern. 2=Prozessierung bei 450°C. 3=Prozessierung bei 450°C+Sputtern.)
   Die XPS-Spektren erlauben eine Aussage über die in der Probe vorhandenen Elemente und der Oxidationsstufe, sowie über das Mengenverhältnis. Damit kann gezeigt werden, dass in den Filmen nach der Prozessierung Magnesiumoxid vorliegt.

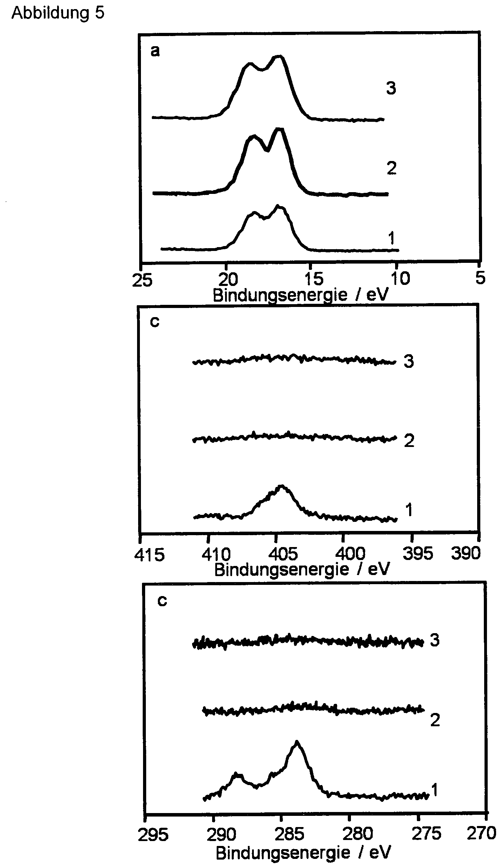

Abb.5: zeigt eine Analyse mittels Röntgenphotonenspektroskopie (XPS) der erfindungsgemäßen Filme aus Hafnium-Oximat in 2-Methoxyethanol/2-Butanol durch Spincoating auf Siliciumsubstraten und Prozessierung bei verschiedenen Temperaturen für jeweils 30 Minuten. (a=Hf4f-Signal. b= N1s-Signal. c=C1s-Signal. 1= ohne Prozessierung. 2=Prozessierung bei 200°C. 3=Prozessierung bei 250°C.)
   Die XPS-Spektren erlauben eine Aussage über die in der Probe vorhandenen Elemente und der Oxidationsstufe, sowie über das Mengenverhältnis. Damit kann gezeigt werden, dass in den Filmen nach der Prozessierung Hafniumoxid vorliegt.

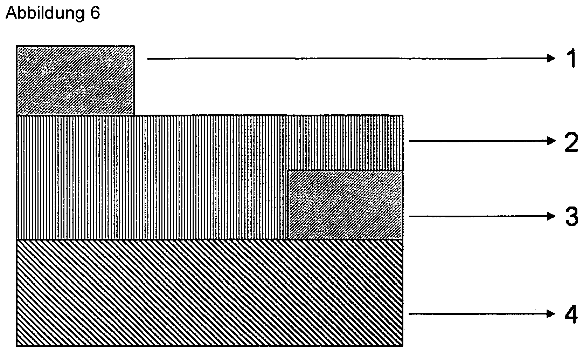

Abb.6: zeigt eine schematische Darstellung des Aufbaus einer erfindungsgemäßen Schicht eines Dielektrikums. (1= Leiter-Gold oder Aluminium, 2 = Dielektrikum-Zirkoniumoxid, 3 = Leiter-Gold oder Aluminium, 4 = Substrat/Isolator-Glas)

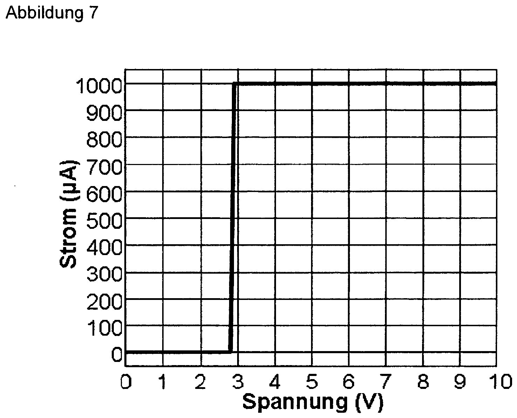

Abb. 7: zeigt eine Strom-Spannungskurve zur Ermittlung der Durchschlagspannung im Bauteil nach Abbildung 6.
   Die Strom-Spannungskurve zeigt den für ein Dielektrikum typischen Verlauf, daraus kann als wichtige Kenngröße des Materials die Durchschlagspannung bestimmt werden.
Abb. 8a: zeigt eine schematische Darstellung des Aufbaus eines Dünnschicht-Feldeffekttransistors (1=Leiter (Drain und Source), 2 =Halbleiter, 3 =Dielektrikum, 4 =Leiter (Gate))
   Das Bauelement weist die Elektroden in Top-Contact Anordnung auf.
Abb. 8b: zeigt eine schematische Darstellung des Aufbaus eines Dünnschicht-Feldeffekttransistors (1 =Halbleiter, 2=Leiter (Drain und Source), 3 = Dielektrikum, 4 = Leiter(Gate))
   Das Bauelement weist die Elektroden in Bottom-Contact Anordnung auf.

## Patentansprüche

1. Precursor zum Beschichten von elektronischen Bauteilen, **dadurch gekennzeichnet, dass** er einen metallorganischen Gallium-, Ruthenium-, Magnesium-, Hafnium-, Indium- und/oder Zinnkomplex sowie deren Gemische enthält, der mindestens einen Liganden aus der Klasse der Oximate enthält.

2. Precursor nach Anspruch 1, **dadurch gekennzeichnet, dass** der Ligand ein 2-(Methoxyimino)alkanoat, 2-(Ethoxyimino)alkanoat oder 2-(Hydroxyimino)alkanoat ist.

3. Gedrucktes, elektronisches Bauelement, welches folgende dünne Schichten aufweist:
• ein festes oder flexibles leitfähiges Substrat oder ein isolierendes Substrat mit einer leitfähigen Schicht (Gate)
• einen Isolator enthaltend ein Aluminium-, Gallium-, Neodym-, Magnesium-, Hafnium- oder Zirkonium-oxid erhältlich aus einem entsprechenden Precursor, der einen metallorganischen Aluminium-, Gallium-, Neodym-, Magnesium-, Hafnium- oder Zirkonium-Komplex sowie deren Gemische enthält, der mindestens einen Liganden aus der Klasse der Oximate enthält,
• mindestens eine Elektrode (Drain-Elektrode)
• einen Halbleiter.

4. Gedrucktes elektronisches Bauelement, welches folgende dünne Schichten aufweist:
• ein festes oder flexibles leitfähiges Substrat oder ein isolierendes Substrat mit einer leitfähigen Schicht (Gate) enthaltend ein Indium-Zinn-oxid (ITO) erhältlich aus dem entsprechenden Precursor nach einem oder mehreren der Ansprüche 1 bis 3.
• einen Isolator
• mindestens eine Elektrode (Drain-Elektrode)
• einen Halbleiter.

5. Gedrucktes elektronisches Bauelement, welches folgende dünne Schichten aufweist:
• ein festes oder flexibles leitfähiges Substrat oder ein isolierendes Substrat mit einer leitfähigen Schicht (Gate)
• einen Isolator
• mindestens eine Elektrode (Drain-Elektrode)
• einen Halbleiter enthaltend ein Indium-Gallium-Zinkoxid (IGZO) oder ein Zink-Zinnoxid (ZTO), erhältlich aus den entsprechenden Precursoren, nach einem oder mehreren der Ansprüche 1 bis 3.

6. Gedrucktes, elektronisches Bauelement nach Anspruch 3, 4 und/oder 5, **dadurch gekennzeichnet, dass** das Substrat sowohl ein festes Substrat wie Glas, Keramik, Metall oder Kunststoffsubstrat als auch ein flexibles Substrat insbesondere Kunststofffolie oder Metallfolie sein kann.

7. Verfahren zur Herstellung eines Precursors nach einem oder mehreren der Ansprüche 1 oder 2, **dadurch gekennzeichnet, dass** mindestens eine Oxocarbonsäure mit mindestens einem Hydroxyl- oder Alkylhydroxylamin in Gegenwart einer Base umgesetzt wird und anschließend ein anorganisches Gallium-, Ruthenium-, Magnesium-, Hafnium-, Indium- und/oder Zinn-Salz sowie deren Gemische zugesetzt wird.

8. Verfahren nach Anspruch 7, **dadurch gekennzeichnet, dass** als Oxocarbonsäure, Oxoessigsäure, Oxopropionsäure oder Oxobuttersäure eingesetzt werden.

9. Verfahren zur Herstellung von elektronischen Strukturen mit Aluminium-, Gallium-, Neodym-, Ruthenium-, Magnesium-, Hafnium-, Zirkonium-, Indium- und/oder Zinn-oxid-Schicht oder -Oberfläche sowie deren Gemische, **dadurch gekennzeichnet, dass**
a. Precursor-Lösungen aus einem metallorganischen Aluminium-, Magnesium-, Gallium-, Neodym-, Ruthenium-, Hafnium-, Zirkonium-, Indium- und/oder Zinn-komplex sowie deren Gemische, die mindestens einen Liganden aus der Klasse der Oximate enthalten, schichtartig wahlweise ein- oder mehrfach entsprechend der zu erzielenden elektronischen Struktur durch Dipcoating, Spincoating oder Inkjet-Drucken bzw. Flexo/Tiefdruck auf ein Substrat aufgebracht werden,
b. Tempern oder Trocknen der aufgebrachten Precursor-Schicht an Luft oder Sauerstoff-Atmosphäre unter Bildung einer Aluminiumoxid-, Galliumoxid-, Neodymoxid-, Rutheniumoxid-, Magnesiumoxid-, Hafniumoxid-, Zirkoniumoxid-, Indiumoxid- und/oder Zinnoxid-Schicht oder -Oberfläche sowie deren Gemische,
c. abschließend die aufgebrachte elektronische Struktur mit einer isolierenden Schicht versiegelt werden kann sowie kontaktiert und komplettiert wird.

10. Verfahren nach Anspruch 9, **dadurch gekennzeichnet, dass** die Temperatur beim Tempern T ≥ 80 °C beträgt.

11. Verfahren nach Anspruch 9, **dadurch gekennzeichnet, dass** das Tempern oder Trocknen durch Bestrahlung mit UV-Licht mit Wellenlängen < 400 nm erfolgt.

12. Verfahren nach einem oder mehreren der Ansprüche 9 bis 11, **dadurch gekennzeichnet, dass** die Aluminiumoxid-, Galliumoxid-, Neodymoxid-, Rutheniumoxid-, Magnesiumoxid-, Hafniumoxid- oder Zirkoniumoxid-Schichten zwischen zwei Leitern eine Durchschlagspannung > 0.1 MV/cm aufweisen.

13. Verfahren nach einem oder mehreren der Ansprüche 9 bis 11, **dadurch gekennzeichnet, dass** die Indium-Zinnoxid-Schicht (ITO-Schicht) einen spezifischen Widerstand, bestimmt durch Vierpunktmessung, von < 10⁻³ Ohm·cm aufweist.

14. Verfahren nach einem oder mehreren der Ansprüche 9 bis 11, **dadurch gekennzeichnet, dass** die Indium-Gallium-Zinkoxid-Schichten (IGZO) oder die Zink-Zinnoxid- Schichten (ZTO) eine Ladungsträger-mobilität > 10⁻³ cm²/Vs aufweisen.

15. Verwendung eines Precursors, welcher einen metallorganischen Aluminium-, Gallium-, Neodym-, Ruthenium-, Magnesium-, Hafnium-, Zirkonium-, Indium- und/oder Zinnkomplex sowie deren Gemische enthält, der mindestens einen Liganden aus der Klasse der Oximate enthält, zum Beschichten von elektronischen Bauteilen.

16. Verwendung nach Anspruch 15, **dadurch gekennzeichnet, dass** der Ligand ein 2-(Methoxyimino)alkanoat, 2-(Ethoxyimino)alkanoat oder 2-(Hydroxyimino)alkanoat ist.

17. Verwendung nach Anspruch 15 oder 16, **dadurch gekennzeichnet, dass** der Precursor druckfähig ist und in Form einer Drucktinte oder Druckpaste in einem gedruckten Feldeffekttransistor (FET) eingesetzt wird.

18. Verwendung nach einem oder mehreren der Ansprüche 15 bis 17 zur Herstellung einer oder mehrerer funktioneller Schichten in einem Feldeffekttransistor.

## Claims

1. Precursor for the coating of electronic components, **characterised in that** it comprises an organometallic gallium, ruthenium, magnesium, hafnium, indium and/or tin complex and mixtures thereof which contains at least one ligand from the class of the oximates.

2. Precursor according to Claim 1, **characterised in that** the ligand is a 2-(methoxyimino)alkanoate, 2-(ethoxyimino)alkanoate or 2-(hydroxyimino)alkanoate.

3. Printed electronic component which has the following thin layers:
• a solid or flexible conductive substrate or an insulating substrate having a conductive layer (gate)
• an insulator comprising an aluminium, gallium, neodymium, magnesium, hafnium or zirconium oxide obtainable from a corresponding precursor which contains an organometallic aluminium, gallium, neodymium, magnesium, hafnium or zirconium complex and mixtures thereof which contains at least one ligand from the class of the oximates
• at least one electrode (drain electrode)
• a semiconductor.

4. Printed electronic component which has the following thin layers:
• a solid or flexible conductive substrate or an insulating substrate having a conductive layer (gate) comprising an indium tin oxide (ITO) obtainable from the corresponding precursor according to one or more of Claims 1 to 3
• an insulator
• at least one electrode (drain electrode)
• a semiconductor.

5. Printed electronic component which has the following thin layers:
• a solid or flexible conductive substrate or an insulating substrate having a conductive layer (gate)
• an insulator
• at least one electrode (drain electrode)
• a semiconductor comprising an indium gallium zinc oxide (IGZO) or a zinc tin oxide (ZTO) obtainable from the corresponding precursors according to one or more of Claims 1 to 3.

6. Printed electronic component according to Claim 3, 4 and/or 5, **characterised in that** the substrate can be both a solid substrate, such as glass, ceramic, metal or plastic substrate, and a flexible substrate, in particular plastic film or metal foil.

7. Process for the preparation of a precursor according to one or more of Claims 1 or 2, **characterised in that** at least one oxocarboxylic acid is reacted with at least one hydroxylamine or alkylhydroxylamine in the presence of a base, and an inorganic gallium, ruthenium, magnesium, hafnium, indium and/or tin salt and mixtures thereof is subsequently added.

8. Process according to Claim 7, **characterised in that** the oxocarboxylic acid employed is oxoacetic acid, oxopropionic acid or oxobutyric acid.

9. Process for the production of electronic structures having an aluminium oxide, gallium oxide, neodymium oxide, ruthenium oxide, magnesium oxide, hafnium oxide, zirconium oxide, indium oxide and/or tin oxide layer or surface and mixtures thereof, **characterised in that**
a. precursor solutions comprising an organometallic aluminium, magnesium, gallium, neodymium, ruthenium, hafnium, zirconium, indium and/or tin complex and mixtures thereof which contain at least one ligand from the class of the oximates are applied to a substrate in either one or more layers corresponding to the electronic structure to be achieved by dip coating, spin coating or ink-jet printing or flexographic/gravure printing,
b. heating or drying of the applied precursor layer in air or an oxygen atmosphere with formation of an aluminium oxide, gallium oxide, neodymium oxide, ruthenium oxide, magnesium oxide, hafnium oxide, zirconium oxide, indium oxide and/or tin oxide layer or surface and mixtures thereof,
c. finally the applied electronic structure can be sealed by means of an insulating layer and is provided with contacts and completed.

10. Process according to Claim 9, **characterised in that** the temperature during the heating T is ≥ 80°C.

11. Process according to Claim 9, **characterised in that** the heating or drying is carried out by irradiation with UV light having wavelengths < 400 nm.

12. Process according to one or more of Claims 9 to 11, **characterised in that** the aluminium oxide, gallium oxide, neodymium oxide, ruthenium oxide, magnesium oxide, hafnium oxide or zirconium oxide layers have a breakdown voltage of > 0.1 MV/cm between two conductors.

13. Process according to one or more of Claims 9 to 11, **characterised in that** the indium tin oxide layer (ITO layer) has a specific resistance, determined by four-point measurement, of < 10⁻³ ohm·cm.

14. Process according to one or more of Claims 9 to 11, **characterised in that** the indium gallium zinc oxide (IGZO) layers or the zinc tin oxide (ZTO) layers have a charge-carrier mobility of > 10⁻³ cm²/Vs.

15. Use of a precursor which comprises an organometallic aluminium, gallium, neodymium, ruthenium, magnesium, hafnium, zirconium, indium and/or tin complex and mixtures thereof which contains at least one ligand from the class of the oximates for the coating of electronic components.

16. Use according to Claim 15, **characterised in that** the ligand is a 2-(methoxyimino)alkanoate, 2-(ethoxyimino)alkanoate or 2-(hydroxyimino)alkanoate.

17. Use according to Claim 15 or 16, **characterised in that** the precursor is printable and is employed in the form of a printing ink or printing paste in a printed field-effect transistor (FET).

18. Use according to one or more of Claims 15 to 17 for the production of one or more functional layers in a field-effect transistor.

## Revendications

1. Précurseur pour le revêtement de composants électroniques, **caractérisé en ce qu'**il comprend un complexe organométallique de gallium, de ruthénium, de magnésium, d'hafnium, d'indium et/ou d'étain et des mélanges afférents, lequel complexe contient au moins un ligand pris parmi la classe des oximates.

2. Précurseur selon la revendication 1, **caractérisé en ce que** le ligand est un 2-(méthoxyimino)alkanoate, un 2-(éthoxyimino)alkanoate ou un 2-(hydroxyimino)alkanoate.

3. Composant électronique imprimé, lequel comporte les couches minces qui suivent :
• un substrat conducteur solide ou flexible ou un substrat isolant comportant une couche conductrice (grille),
• un isolant comprenant un oxyde d'aluminium, de gallium, de néodyme, de magnésium, d'hafnium ou de zirconium qui peut être obtenu à partir d'un précurseur correspondant, lequel précurseur contient un complexe organométallique d'aluminium, de gallium, de néodyme, de magnésium, d'hafnium ou de zirconium et des mélanges afférents, lequel complexe contient au moins un ligand pris parmi la classe des oximates,
• au moins une électrode (électrode de drain),
• un semiconducteur.

4. Composant électronique imprimé, lequel comporte les couches minces qui suivent :
• un substrat conducteur solide ou flexible ou un substrat isolant comportant une couche conductrice (grille) comprenant un oxyde d'indium et d'étain (ITO) qui peut être obtenu à partir du précurseur correspondant selon une ou plusieurs des revendications 1 à 3,
• un isolant,
• au moins une électrode (électrode de drain),
• un semiconducteur.

5. Composant électronique imprimé, lequel comporte les couches minces qui suivent :
• un substrat conducteur solide ou flexible ou un substrat isolant comportant une couche conductrice (grille),
• un isolant,
• au moins une électrode (électrode de drain)
• un semiconducteur comprenant un oxyde d'indium, de gallium et de zinc (IGZO) ou un oxyde de zinc et d'étain (ZTO) qui peut être obtenu à partir des précurseurs correspondants selon une ou plusieurs des revendications 1 à 3.

6. Composant électronique imprimé selon la/les revendication(s) 3, 4 et/ou 5, **caractérisé en ce que** le substrat peut être à la fois un substrat solide, tel qu'un substrat en verre, en céramique, en métal ou en matière plastique, et un substrat flexible, en particulier un film en matière plastique ou une feuille en métal.

7. Procédé pour la préparation d'un précurseur selon une ou plusieurs des revendications 1 ou 2, **caractérisé en ce qu'**au moins un acide oxocarboxylique est amené à réagir avec au moins un hydroxylamine ou un alkylhydroxylamine en présence d'une base, et un sel inorganique de gallium, de ruthénium, de magnésium, d'hafnium, d'indium et/ou d'étain et des mélanges afférents est ensuite ajouté.

8. Procédé selon la revendication 7, **caractérisé en ce que** l'acide oxocarboxylique utilisé est de l'acide oxoacétique, de l'acide oxopropionique ou de l'acide oxobutyrique.

9. Procédé pour la production de structures électroniques comportant une couche ou surface en oxyde d'aluminium, en oxyde de gallium, en oxyde de néodyme, en oxyde de ruthénium, en oxyde de magnésium, en oxyde d'hafnium, en oxyde de zirconium, en oxyde d'indium et/ou en oxyde d'étain et des mélanges afférents, **caractérisé en ce que** :
a. des solutions de précurseur comprenant un complexe organométallique d'aluminium, de magnésium, de gallium, de néodyme, de ruthénium, d'hafnium, de zirconium, d'indium et/ou d'étain et des mélanges afférents, lesquelles contiennent au moins un ligand pris parmi la classe des oximates, sont appliquées sur un substrat selon soit une couche, soit plusieurs couches en correspondance avec la structure électronique destinée à être obtenue par revêtement/ dépôt par trempage/immersion, dépôt à la tournette/par centrifugation ou impression par jet d'encre ou impression flexographique/par gravure,
b. la couche de précurseur appliquée est chauffée ou séchée dans l'air ou dans une atmosphère d'oxygène avec la formation d'une couche ou surface en oxyde d'aluminium, en oxyde de gallium, en oxyde de néodyme, en oxyde de ruthénium, en oxyde de magnésium, en oxyde d'hafnium, en oxyde de zirconium, en oxyde, d'indium et/ou en oxyde d'étain et des mélanges afférents,
c. pour finir, la structure électronique résultant de l'application peut être scellée/rendue étanche au moyen d'une couche isolante et elle est munie de contacts et parachevée.

10. Procédé selon la revendication 9, **caractérisé en ce que** la température pendant le chauffage T est ≥ 80°C.

11. Procédé selon la revendication 9, **caractérisé en ce que** le chauffage ou le séchage est mis en oeuvre par irradiation avec une lumière UV présentant des longueurs d'onde < 400 nm.

12. Procédé selon une ou plusieurs des revendications 9 à 11, **caractérisé en ce que** les couches en oxyde d'aluminium, en oxyde de gallium, en oxyde de néodyme, en oxyde de ruthénium, en oxyde de magnésium, en oxyde d'hafnium ou en oxyde de zirconium présentent une tension de rupture/claquage > 0,1 MV/cm entre deux conducteurs.

13. Procédé selon une ou plusieurs des revendications 9 à 11, **caractérisé en ce que** la couche en oxyde d'indium et d'étain (couche en ITO) présente une résistance spécifique, déterminée au moyen d'une mesure en quatre points, < 10⁻³ ohm·cm.

14. Procédé selon une ou plusieurs des revendications 9 à 11, **caractérisé en ce que** les couches en oxyde d'indium, de gallium et de zinc (IGZO) ou les couches en oxyde de zinc et d'étain (ZTO) présentent une mobilité de porteurs de charges > 10⁻³ cm²/Vs.

15. Utilisation d'un précurseur, lequel comprend un complexe organométallique d'aluminium, de gallium, de néodyme, de ruthénium, de magnésium, d'hafnium, de zirconium, d'indium et/ou d'étain et des mélanges afférents, lequel complexe contient au moins un ligand pris parmi la classe des oximates pour le revêtement de composants électroniques.

16. Utilisation selon la revendication 15, **caractérisée en ce que** le ligand est un 2-(méthoxyimino)alkanoate, un 2-(éthoxyimino)alkanoate ou un 2-(hydroxyimino)alkanoate.

17. Utilisation selon la revendication 15 ou 16, **caractérisée en ce que** le précurseur est imprimable et est utilisé sous la forme d'une encre d'impression ou d'une pâte d'impression dans un transistor à effet de champ (FET) imprimé.

18. Utilisation selon une ou plusieurs des revendications 15 à 17 pour la production d'une ou de plusieurs couche(s) fonctionnelle(s) dans un transistor à effet de champ.
